# EUROPEAN PATENT APPLICATION

(11) **EP 3 845 246 A1**
(43) Date of publication of application: **07.07.2021**
(21) Application number: 19855524.5
(22) Date of filing: 02.09.2019
(51) Int. Cl.: A61K 45/00, A61K 31/7088, A61K 39/395, A61K 48/00, A61P 1/04, A61P 9/00, A61P 25/00, G01N 33/15

(54) **MEDICINE FOR PREVENTING AND/OR TREATING STRESS LOAD-RELATED DISEASE**

(30) Priority: 01.09.2018 JP 2018164092
(71) Applicant: National University Corporation Hokkaido University, Sapporo-shi, Hokkaido 060-0808 (JP)
(72) Inventor: MURAKAMI Masaaki, Sapporo-shi, Hokkaido 060-0808 (JP); KAMIMURA Daisuke, Sapporo-shi, Hokkaido 060-0808 (JP)
(74) Representative: Wasner, Marita
(86) International application number: PCT/JP2019/034497
(87) International publication number: WO 2020/045683

(57) **Abstract**

The present invention pertains to a medicine, said medicine comprising as an active ingredient a substance which suppresses the expression of at least one molecule selected from the group consisting of TMEM5, VSTM2L, C2CD4D, VSTM2A, LY6G6C and ADRA2C or inhibits the activity thereof, for preventing and/or treating at least one disease or symptom selected from the group consisting of progressive multiple sclerosis, gastroenteritis, myocardial disorder and sudden death. Also, the present invention pertains to a method for screening a substance, which is capable of preventing and/or treating at least one disease or symptom selected from the group consisting of progressive multiple sclerosis, gastroenteritis, myocardial disorder and sudden death, with the use of the expression or activity of the aforesaid molecule as an index.

## Description

### Field

The present invention relates to a pharmaceutical for preventing and/or treating a stress load-related disease or symptom, such as progressive multiple sclerosis, gastroenteritis, myocardial disorder, or sudden death, and relates to a screening method.

### Background

Various external factors, such as noise, chill, drugs, bacterial infection, and furthermore human relations and work accountability, are generally referred to as stress. Stress is empirically known to cause non-specific abnormal conditions to human body. For example, stress can damage mental and physical homeostasis maintenance functions, and bring relatively mild diseases or symptoms, such as asthma, spot baldness, urinary frequency, tinnitus, and dizziness.

Stress can furthermore induce serious diseases or symptoms, for example, neurotic diseases, such as depression, panic disorder, and anxiety disorder, gastrointestinal ulceration, irritable bowel syndrome, and ischemic cardiac diseases, and sometimes stress can induce directly life-threatening symptoms, for example, sudden death. Sudden death is defined as natural death that occurs within 24 hours from the onset, and a typical example thereof is cardiac sudden death caused by cardiac diseases.

In order to investigate effective preventing or treating methods for various diseases caused by stress, medical researches on associations between stress and various diseases or symptoms are being pursued. For example, stress is known to cause a gastrointestinal disease via brain-gut interaction. Through a research using a model animal, it was reported that brain-gut interaction is involved with interactions among nerve-constituting elements, for example, the autonomic nerve system, the central nerve system, and stress systems such as the hypothalamic-pituitary-adrenal axis, and the corticotropin-releasing factor system, and gut factors, such as intestinal barrier, luminal microbiota, and intestinal immune response (Non Patent literature 1).

Moreover, through molecular-biological researches on the association of stress with various diseases or symptoms, it is being elucidated that stress hormones, typified by corticotropin-releasing hormone (CRH), neurotransmitters, such as noradrenaline, serotonin and dopamine, and other various neuropeptides are involved in biological responses to stress.

By using an animal model of multiple sclerosis (experimental autoimmune encephalomyelitis, EAE model), the present inventors investigated a process from a pain stimulus to the onset of a symptom of multiple sclerosis, and found that the symptom of multiple sclerosis develops through the steps (gateway reflex) of activation of sensory nerves by pain, activation of sympathetic nerves, infiltration of immune cells into ventral vessels in the fifth lumbar cord, and activation of the inflammation amplifier by the infiltration (Non Patent Literature 2). This study results advocate that suppressing the neural network originating from pain, for example, administrating an analgesic agent, can provide new means for not only removing the pain but also preventing the relapse of multiple sclerosis.

Furthermore, the present inventors found a new gateway reflex in which CD4 positive T cells reactive to an antigen derived from a central nervous tissue are transferred to a chronically stressed mouse, whereby microscopic inflammation is induced at specific cerebral blood vessels, and reported the possibility of this gateway reflex inducing various diseases or symptoms, such as multiple sclerosis, gastroenteritis, myocardial disorder, and sudden death (Non Patent Literature 3). A blockade of this gateway reflex has become a focus of attention as a new approach for preventing and treating the above-mentioned various diseases or symptoms.

### Citation List

### Non Patent Literature

Non Patent Literature 1: Non Patent Literature 1: Caso, J. R. et al., Current molecular medicine, 2008, Vol. 8, pp. 299-312
Non Patent Literature 2: Arima, Y. et al., eLife, 2015, 4: e08733
Non Patent Literature 3: Arima, Y. et al., eLife, 2017, 6: e25517

### Summary

### Technical Problem

An object of the present invention is to provide a novel pharmaceutical for preventing and/or treating a stress load-related disease or symptom, especially, a chronic stress load-related disease or symptom.

### Solution to Problem

The present inventors investigated a phenomenon in which inflammation at specific cerebral blood vessels due to stress load induces the above-mentioned diseases or symptoms, and found that the expression of Transmembrane Protein 5 (TMEM5), V-Set And Transmembrane Domain Containing 2 Like (VSTM2L), C2 Calcium Dependent Domain Containing 4D (C2CD4D), V-Set And Transmembrane Domain Containing 2A (VSTM2A), lymphocyte antigen 6 family member G6C (LY6G6C), and Adrenoceptor Alpha 2C (α2C adrenergic receptor, ADRA2C) is enhanced at the blood vessels, and thus the present inventors completed the following invention.

(1) A pharmaceutical for preventing and/or treating at least one disease or symptom selected from the group consisting of progressive multiple sclerosis, gastroenteritis, myocardial disorder, and sudden death, the pharmaceutical containing, as an active ingredient, a substance that suppresses the expression of or inhibits the activity of at least one molecule selected from the group consisting of TMEM5, VSTM2L, C2CD4D, and VSTM2A, the substance being at least one selected from the group consisting of a nucleic acid, an antibody, and an antibody derivative.
(2) A pharmaceutical for preventing and/or treating at least one disease or symptom selected from the group consisting of progressive multiple sclerosis, gastroenteritis, myocardial disorder, and sudden death, the pharmaceutical containing, as an active ingredient, a nucleic acid that suppresses the expression of at least one molecule selected from the group consisting of LY6G6C and ADRA2C.
(3) The pharmaceutical according to (1) or (2), wherein the disease or symptom is a disease or symptom caused by stress load.
(4) A method for screening a substance capable of preventing and/or treating at least one disease or symptom selected from the group consisting of progressive multiple sclerosis, gastroenteritis, myocardial disorder, and sudden death, the method including the steps of: (i) in a cell or an animal that expresses at least one molecule selected from the group consisting of TMEM5, VSTM2L, C2CD4D, VSTM2A, LY6G6C, and ADRA2C, measuring the expression level or activity of the molecule in the presence and absence of a test substance; and (ii) when the expression level or activity of the molecule in the presence of the test substance is lower or weaker than the expression level or activity of the molecule in the absence of the test substance, determining that the test substance has the ability to suppress the expression of or inhibit the activity of the molecule.

### Advantageous Effects of Invention

By using the pharmaceutical according to the present invention, at least one disease or symptom selected from the group consisting of progressive multiple sclerosis, gastroenteritis, myocardial disorder, and sudden death can be prevented and/or treated.

### Brief Description of Drawings

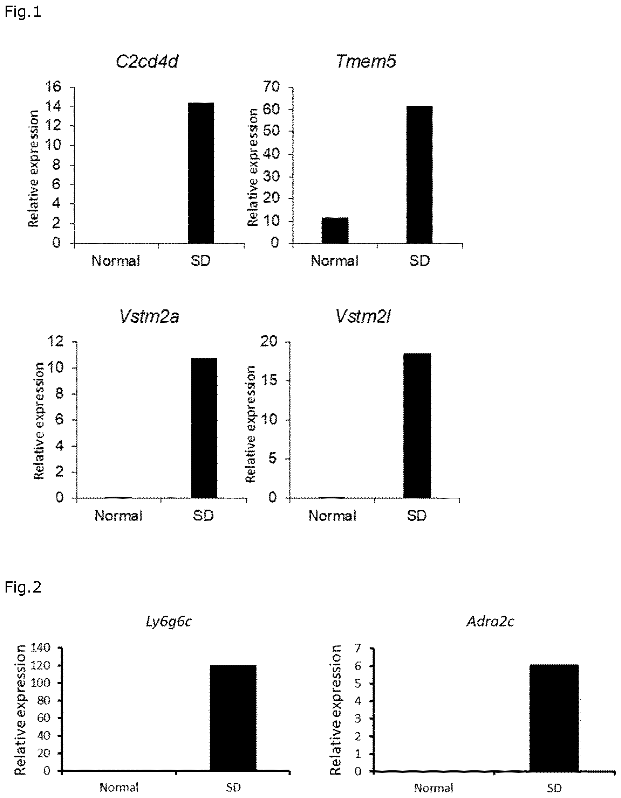

Fig. 1 includes graphs illustrating the gene expression levels of TMEM5, VSTM2L, C2CD4D, and VSTM2A at specific blood vessels of mice in which sleep disorder (SD) was induced.
Fig. 2 includes graphs illustrating the gene expression levels of LY6G6C and ADRA2C at specific blood vessels of mice in which sleep disorder (SD) was induced.

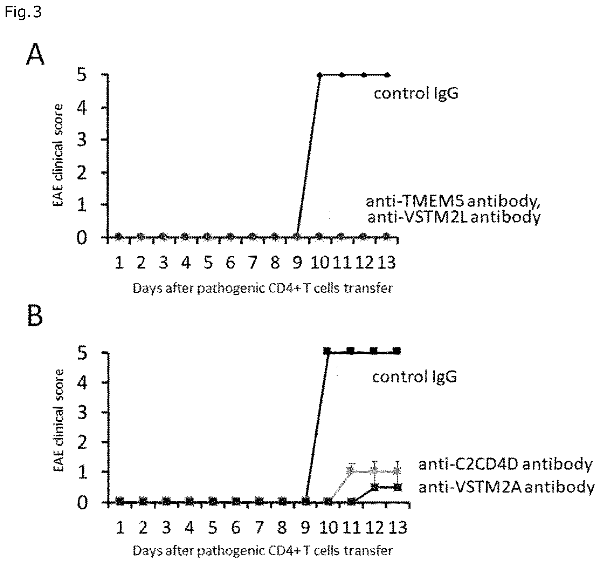

Fig. 3 includes graphs illustrating pathological conditions of mice in which sleep disorder was induced, then to which EAE-pathogenic CD4 positive T cells were transferred, and subsequently to which an anti-TMEM5 antibody, an anti-VSTM2L antibody, an anti-C2CD4D antibody, or an anti-VSTM2A antibody was microinjected. Fig. 3A illustrates changes over time of EAE clinical scores of the mice to which the anti-TMEM5 antibody or the anti-VSTM2L antibody was administered. Fig. 3B illustrates changes over time of EAE clinical scores of the mice to which the anti-C2CD4D antibody or the anti-VSTM2A antibody was administered.

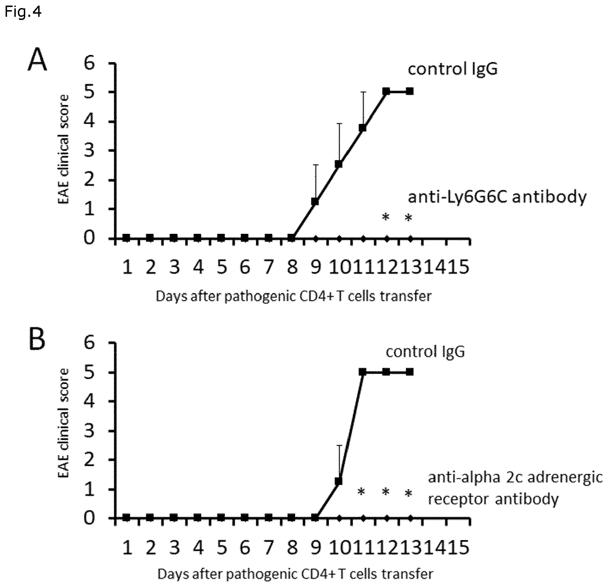

Fig. 4 includes graphs illustrating pathological conditions of mice in which sleep disorder was induced, then to which EAE-pathogenic CD4 positive T cells were transferred, and subsequently to which an anti-LY6G6C antibody or an anti-ADRA2C antibody was microinjected. Fig. 4A illustrates changes over time of EAE clinical scores of the mice to which the anti-LY6G6C antibody was administered. Fig. 4B illustrates changes over time of EAE clinical scores of the mice to which the anti-ADRA2C antibody was administered.

### Description of Embodiments

### Pharmaceutical for preventing and/or treating disease or symptom

A first aspect of the present invention relates to a pharmaceutical for preventing and/or treating at least one disease or symptom selected from the group consisting of progressive multiple sclerosis, gastroenteritis, myocardial disorder, and sudden death, the pharmaceutical containing, as an active ingredient, a substance that suppresses the expression of or inhibits the activity of at least one molecule selected from the group consisting of TMEM5, VSTM2L, C2CD4D, VSTM2A, LY6G6C, and ADRA2C.

In a preferable embodiment, the present invention provides a pharmaceutical for preventing and/or treating at least one disease or symptom selected from the group consisting of progressive multiple sclerosis, gastroenteritis, myocardial disorder, and sudden death, the pharmaceutical containing, as an active ingredient, a substance that suppresses the expression of or inhibits the activity of at least one molecule selected from the group consisting of TMEM5, VSTM2L, C2CD4D, and VSTM2A.

In another preferable embodiment, the present invention provides a pharmaceutical for preventing and/or treating at least one disease or symptom selected from the group consisting of progressive multiple sclerosis, gastroenteritis, myocardial disorder, and sudden death, the pharmaceutical containing, as an active ingredient, a nucleic acid that suppresses the expression of at least one molecule selected from the group consisting of LY6G6C and ADRA2C.

The present inventors investigated a phenomenon in which inflammation at specific cerebral blood vessels due to stress load induces the above-mentioned diseases or symptoms, and found the following mechanism. In a living body having been stressed and fallen into a stress condition, sympathetic nerves are activated in its paraventricular hypothalamic nucleus (PVN), and noradrenaline is secreted from blood vessels present in the boundary region of the third ventricle, thalamus, and dentate gyrus. Then, at the blood vessels, the production of, for example, CCL5 is enhanced, CD11b positive MHC class II-highly expressing cells and CD4 positive T cells are accumulated, and the production and augmentation of inflammatory cytokines are enhanced, whereby inflammation is induced. The inflammation at the blood vessels activates nerves in the dorsal medial hypothalamic nucleus (DMH) through ATP, and furthermore, this activation induces various diseases, such as progressive multiple sclerosis, gastroenteritis, myocardial disorder, and sudden death through the activation of the vagal nerves in the dorsal vagal nucleus (DMX) and nucleus tractus solitarii (NTS) (refer to PCT/JP2018/007901 and Arima, Y. et al., eLife, 2017, 6: e25517, the total contents of which are to be incorporated herein by reference).

As described in the later-mentioned Examples, the present inventors furthermore found that stress load causes the expression of TMEM5, VSTM2L, C2CD4D, VSTM2A, LY6G6C, and ADRA2C to be enhanced at specific blood vessels present in the boundary region of the third ventricle, thalamus, and dentate gyrus, and the administration of antibodies against them to the blood vessels suppresses the occurrence or progression of pathological conditions in the disease modeling animal. While not wishing to be bound by any theory, since these antibodies exert effects when directly administered to the blood vessels, these antibodies are presumed to inhibit the occurrence of reactions at the specific blood vessels among a series of reactions that start from stress load, through the induction of inflammation at the specific blood vessels, leading to the occurrence of pathological conditions.

Based on the above-described mechanism, inhibiting the functions of TMEM5, VSTM2L, C2CD4D, VSTM2A, LY6G6C, or ADRA2C is considered to work for preventing and/or treating various diseases or symptoms exhibited in the disease modeling animal. Therefore, a substance and a method that are configured to bring such inhibition of the functions are expected to be effective for preventing and/or treating stress load-related diseases, especially, progressive multiple sclerosis, gastroenteritis, myocardial disorder, and sudden death, which are caused by stress load.

It has been further revealed that various diseases or symptoms induced by the above-described mechanism are mutually related, and preventing and/or treating one disease or symptom contribute to preventing and/or treating another disease or symptom (see PCT/JP2018/007901 and Arima, Y. et al., eLife, 2017, 6: e25517). Accordingly, a substance or method for preventing and/or treating at least one disease or symptom selected from the group consisting of progressive multiple sclerosis, gastroenteritis, myocardial disorder, and sudden death, in a subject with cerebrovascular inflammation, is expected to be effective for preventing and/or treating the other diseases or symptoms included in the group described above.

### Active ingredient of pharmaceutical

The pharmaceutical according to the present invention contains, as an active ingredient, a substance that suppresses the expression of or inhibits the activity of at least one molecule selected from the group consisting of TMEM5, VSTM2L, C2CD4D, VSTM2A, LY6G6C, and ADRA2C. The expression "containing, as an active ingredient," used herein means containing an effective amount of a substance or containing a combination of such substance and others. The effective amount is appropriately determined by those skilled in the art, depending on the kind or use of the substance, the age, sex, or weight of a subject, the kind or severity of a disease or symptom, or other conditions.

TMEM5 (Transmembrane Protein 5, also known as Ribitol Xylosyltransferase 1) is a type-II transmembrane protein presumed to be glycosyltransferase. The nucleotide sequences of the genes of human TMEM5 are registered as XM_005268562.3, XM_017018686.1, XM_017018687.1, and XM_005268563.3 in the GenBank, and the amino acid sequences thereof are registered as XP_005268619.1, XP_016874175.1, XP_016874176.1, and XP_005268620.1 in the GenBank.

VSTM2L (V-Set And Transmembrane Domain Containing 2 Like, also known as C20orf102) is known as a secretory antagonist that regulates the action of a neuroprotective peptide, namely, humanin. The nucleotide sequence of the gene of human VSTM2L is registered as NM_080607.2 in the GenBank, and the amino acid sequence thereof is registered as NP_542174.1 in the GenBank.

C2CD4D (C2 Calcium Dependent Domain Containing 4D) is a protein having an unknown function. The nucleotide sequence of the gene of human C2CD4D is registered as NM_001136003.1 in the GenBank, and the amino acid sequence thereof is registered as NP_001129475.1 in the GenBank.

VSTM2A (V-Set And Transmembrane Domain Containing 2A) is a protein secreted in adipose precursor cells, and is known to function at the early stage of their differentiation. The nucleotide sequences of the genes of human VSTM2A are registered as NM_001301009.1, NM_001317843.1, and NM_182546.3 in the GenBank, and the amino acid sequences thereof are registered as NP_001287938.1, NP_001304772.1, and NP_872352.2 in the GenBank.

LY6G6C (lymphocyte antigen 6 family member G6C) is a lymphocyte antigen-6 (LY6) family member positioned in the region of major histocompatibility complex (MHC) class III of chromosome 6. The nucleotide sequence of the gene of human LY6G6C is registered as NM_025261.2 in the GenBank, and the amino acid sequence thereof is registered as NP_079537.1 in the GenBank.

ADRA2C (α2C adrenergic receptor, Adrenoceptor Alpha 2C) is one subtype of alpha-2-adrenergic receptor, which is a member of the G protein-coupled receptor super-family. The nucleotide sequence of the gene of human ADRA2C is registered as NM_000683.3 in the GenBank, and the amino acid sequence thereof is registered as NP_000674.2 in the GenBank.

Examples of the substance that suppresses the expression of at least one molecule (also referred to as "target molecule") selected from the group consisting of TMEM5, VSTM2L, C2CD4D, VSTM2A, LY6G6C, and ADRA2C include a substance capable of suppressing transcription of mRNA from genes encoding these molecules, a substance capable of degrading the mRNA, and a substance capable of suppressing protein translation from the mRNA. Typical examples of these substances include inhibitory nucleic acids, such as antisense RNA and siRNA, that those skilled in the art can design and produce, based on the nucleotide sequences of genes encoding these molecules and mRNA to be translated into protein. Examples of the inhibitory nucleic acids that are commercially available may include: as for TMEM5, SMARTpool: Accell TMEM5 siRNA (E-020635-00); as for VSTM2L, SMARTpool: Accell VSTM2L siRNA (E-015237-00); as for C2CD4D, SMARTpool: Accell C2CD4D siRNA (SMARTpool: Accell C2CD4D siRNA); as for VSTM2A, SMARTpool: Accell VSTM2A siRNA (E-018827-01); as for LY6G6C, SMARTpool: Accell LY6G6C siRNA (E-014622-00); and, as for ADRA2C, SMARTpool: Accell ADRA2C siRNA (E-005424-00) (all of them are manufactured by Dharmacon, Inc). The above-mentioned products each contain four kinds of siRNA to their targets, and in the present invention, one of the four kinds of siRNA may be used alone, or two or more kinds thereof may be used in combination.

Furthermore, shRNA containing the nucleotide sequence of siRNA, DNA capable of inducing the transcription of, for example, the antisense RNA or the siRNA when placed under the control of a suitable expression promotor, and RNA having enhanced stability against degradation by nuclease by modifying a part of nucleotide sequences of, for example, the antisense RNA or the siRNA are also encompassed by the substance that suppresses the expression according to the present invention, as a nucleic acid functionally equivalent to the antisense RNA or the siRNA. Examples of the modification for enhancing stability against degradation by nuclease include 2'O-methylation, 2'-fluorination, and 4'-thiolation.

Furthermore, chimeric RNA in which a part of the ribonucleotide of the above-mentioned RNA is replaced by the corresponding deoxyribonucleotide or nucleotide analog is also encompassed by the substance that suppresses the expression according to the present invention. Examples of the nucleotide analog may include 5-modified uridine or cytidine, such as 5-(2-amino)propyl uridine and 5-bromouridine; 8-modified adenosine or guanosine, such as 8-bromoguanosine; deaza-nucleotide, such as 7-deaza-adenosine; and O- or N-alkylated nucleotide, such as N6-methyladenosine.

The kind or the number of bases modified or replaced in the above-mentioned inhibitory nucleic acid are not particularly limited unless the ability to suppress the expression of a target molecule is lost.

The above-mentioned inhibitory nucleic acid can be artificially synthesized by utilizing a genetic recombination technique or a chemical synthesis technique. As a method of genetic recombination, a method of chemically synthesizing a nucleic acid, a method of synthesizing a non-natural base, or a method of synthesizing a nucleic acid containing a non-natural base, a method well known to those skilled in the art can be employed. A nucleic acid may be synthesized by using an apparatus, such as what is called a DNA synthesizer.

Examples of the substance that inhibits the activity of at least one molecule selected from the group consisting of TMEM5, VSTM2L, C2CD4D, VSTM2A, LY6G6C, and ADRA2C may include low molecular compounds that bind to these molecules to inhibit the activity of the molecules, neutralizing antibodies that specifically bind to these molecules, and antibody derivatives that specifically bind to these molecules. The above-mentioned antibodies can be monoclonal antibodies, chimeric antibodies, humanized antibodies, or human antibodies. The antibody derivatives can be antibody fragments, such as Fab, Fab', F(ab')2, scFv, or F(ab')2, or diabody, dsFv, or peptides containing CDRs, derived from the antibodies.

The antibodies can be prepared by a common method for producing an antibody, the method including immunizing a suitable laboratory animal such as a rabbit, a mouse, or a rat by using a target molecule as an antigen, the target molecule being preferably produced by a genetic recombination technique. Alternatively, commercially available antibodies, such as a rabbit anti-TMEM5 antibody (Novus (NB110-40445), Anti-TMEM5, rabbit Poly), a mouse anti-VSTM2L antibody (Santa Cruz Biotechnology (SC-376538) Anti VSTM2L (A-4), monoclonal IgG1), a rabbit anti-C2CD4D antibody (Bioss (bs-15144R), AntiC2CD4D Polyclonal Antibody), a rabbit anti-VSTM2A antibody (Thermo Fisher (PA5-48140), VSTM2A Polyclonal Antibody), a rat anti-Ly6G6C antibody (clone; NIMP-R14, monoclonal IgG2b (NOVUS BIOLOGICALS)), a rat anti-Ly6C6G antibody (LS-C663018, polyclonal, LifeSpan BioSciences), and a rabbit anti-alpha 2c Adrenergic Receptor antibody (polyclonal, GeneTex) may be used, or alternatively, antibodies containing the same CDR sequences as the CDR sequences of these antibodies may be used.

The pharmaceutical according to the present invention may contain a substance capable of suppressing the expression of or inhibiting the activity of a target molecule, other than the above-mentioned nucleic acids, antibodies, and antibody derivatives. Such substance can be searched through screening for the ability to suppress the expression or the ability to inhibit the activity by using a target molecule or a suitable cell or animal that expresses the target molecule.

### Screening Method

Another aspect of the present invention relates to a method for screening a substance capable of suppressing the expression of or inhibiting the activity of of at least one molecule selected from the group consisting of TMEM5, VSTM2L, C2CD4D, and VSTM2A, in other words, a substance capable of preventing and/or treating a disease or symptom caused by stress load, especially by chronic stress load, typically, at least one disease or symptom selected from the group consisting of progressive multiple sclerosis, gastroenteritis, myocardial disorder, and sudden death, the method including the steps of: (i) in a cell or an animal that expresses at least one molecule selected from the group consisting of TMEM5, VSTM2L, C2CD4D, VSTM2A, LY6G6C, and ADRA2C, measuring the expression level or activity of the molecule in the presence and absence of a test substance; and (ii) when the expression level or activity of the molecule in the presence of the test substance is lower or weaker than the expression level or activity of the molecule in the absence of the test substance, determining that the test substance has the ability to suppress the expression of or inhibit the activity of the molecule, in other words, the test substance is capable of preventing and/or treating a disease or symptom caused by stress load, especially by chronic stress load, typically, at least one disease or symptom selected from the group consisting of progressive multiple sclerosis, gastroenteritis, myocardial disorder, and sudden death.

The cell or the animal, used at the step (i), that expresses at least one molecule selected from the group consisting of TMEM5, VSTM2L, C2CD4D, VSTM2A, LY6G6C, and ADRA2C may be a naturally present cell or animal or may be a cell or an animal modified so as to express the molecule. One example of the cell or the animal is a cell into which a gene encoding the molecule is integrated to forcibly express the molecule. Another example of the cell or the animal is a disease modeling non-human animal described in PCT/JP2018/007901 and Arima, Y. et al., eLife, 2017, 6: e25517, that is produced by causing CD4 positive T cells reactive to an antigen derived from the central nervous tissue to be present in the body of a non-human animal in a stress condition and has inflammation at a blood vessel in the boundary region of the third ventricle, thalamus, and dentate gyrus.

The measurement of the expression level or activity of at least one molecule selected from the group consisting of TMEM5, VSTM2L, C2CD4D, VSTM2A, LY6G6C, and ADRA2C at the step (i) can be performed using a cell that expresses the molecule after the cell is put in a physiologically acceptable liquid, such as a culture medium or buffer solution, containing the test substance. Alternatively, the measurement of the expression level or activity of the molecule can be performed by collecting perivascular tissue in the boundary region of the third ventricle, thalamus, and dentate gyrus from an animal that expresses the molecule after the test substance is administered thereto, and using the perivascular tissue.

The expression level of at least one molecule selected from the group consisting of TMEM5, VSTM2L, C2CD4D, VSTM2A, LY6G6C, and ADRA2C is the protein expression level of the molecule or the expression level of mRNA encoding the molecule, and these expression levels can be measured by employing various methods known to those skilled in the art, the methods being capable of confirming the expression of a protein or gene having a known amino acid sequence or nucleotide sequence.

The measurement of the protein expression level of the molecule can be performed using an antibody specific thereto by a known method, such as enzyme-linked immunosorbent assay (ELISA) such as direct ELISA, indirect ELISA, or sandwich ELISA, radioimmunoassay (RIA), in situ hybridization, immunoblot analysis, western blot analysis, or tissue array analysis.

The measurement of the expression level of mRNA encoding the molecule can be performed by a known method capable of detecting the expression of mRNA, such as a PCR method using a primer nucleic acid having a suitable nucleotide sequence designed based on the nucleotide sequence of mRNA; a hybridization method using a probe nucleic acid having a nucleotide sequence capable of being hybridized under stringent conditions with the nucleotide sequence of mRNA, or DNA or RNA (cDNA or cRNA) complementary to the nucleotide sequence of mRNA; a microarray method using a chip to which a probe nucleic acid having a nucleotide sequence capable of being hybridized with the nucleotide sequence of mRNA is fixed; or a RNA sequencing method.

The activity of at least one molecule selected from the group consisting of TMEM5, VSTM2L, C2CD4D, VSTM2A, LY6G6C, and ADRA2C can be measured using systems in accordance with the respective physiological functions of the molecules. The activity can be measured, for example, using, as an indicator, a quantitative or qualitative change in substrate caused by an enzyme reaction when the molecule is an enzyme, or using, as an indicator, a quantitative or qualitative change in protein present downstream of signal transduction pathway that is caused by ligand-receptor interaction when the molecule is a receptor or ligand.

### Pharmaceutical Preparation

The pharmaceutical according to the present invention can be used in the form of a pharmaceutical composition containing the above-mentioned active ingredients as well as drugs other than the active ingredients, or pharmaceutically acceptable ingredients, such as a buffer, an anti-oxidant, a preservative, protein, a hydrophilic polymer, amino acid, a chelator, a nonionic surfactant, an excipient, a stabilizer, and a carrier, or used as a pharmaceutical preparation containing them. Such pharmaceutical composition is also encompassed by the pharmaceutical according to the present invention. The pharmaceutically acceptable ingredients are well-known to those skilled in the art, and can be appropriately selected, for example, from ingredients described in the Japanese Pharmacopoeia, 17th Edition or other written standards, depending on the form of the pharmaceutical preparation, and is used by those skilled in the art, within the scope of their ordinary implementation ability.

The pharmaceutical according to the present invention can be in any form, and the form can be suitably selected in accordance with, for example, a target site or the kind of a disease or symptom. The pharmaceutical is typically preferably in the form of a parenteral formulation, such as an injection, a percutaneous formulation, an enteral formulation, or a drip. The administration route for the pharmaceutical according to the present invention is not particularly limited, and when the pharmaceutical is a parenteral formulation, examples of the administration route include intravascular administration (preferably, intravenous administration), intraperitoneal administration, intestinal administration, subcutaneous administration, and topical administration to a target site. In one preferable embodiment, the pharmaceutical according to the present invention is administered to a living body by intravenous administration or topical administration.

The dosage of the pharmaceutical according to the present invention is appropriately selected by those skilled in the art, depending on the use of the pharmaceutical, the age, sex, or weight of a subject, the kind or severity of a disease or symptom, or other conditions. The pharmaceutical according to the present invention may be used in combination with another pharmaceutical effective in preventing and/or treating progressive multiple sclerosis, gastroenteritis, myocardial disorder, or sudden death.

### Use of Pharmaceutical

The pharmaceutical according to the present invention is useful for preventing and/or treating a disease or symptom caused by stress load, especially by chronic stress load, and typically can be used for preventing and/or treating at least one disease or symptom selected from the group consisting of progressive multiple sclerosis, gastroenteritis, myocardial disorder, and sudden death. The disease or symptom can also be expressed as a disease or symptom that a subject having been in a stress condition or being in a stress condition is affected with or develops or has the risk of being affected with or developing.

A medical term "stress" refers to both an adverse factor (the cause of stress, stressor) brought from the outside of the body and a defense reaction resulted from the adverse factor (Nanzando's Medical Dictionary, 19th Edition). In the present specification, the adverse factor is referred to as stress, and exposure to the adverse factor is referred to as being stressed or being loaded with stress, while the defense reaction is referred to as a stress reaction. Examples of the stress as the adverse factor include physical stress (such as chill, noise, and radiation), chemical stress (such as oxygen and drugs), biological stress (such as inflammation and infection), and mental stress (such as anger and anxiety), and, generally, mental stress is regarded as a problem for humans because of its adverse influence on human health.

According to what is called stress theory, the stress reaction is a series of reactions called adaptation syndrome that a living body causes to defense itself from stress. The stress reaction can be divided into three stages: an alarm reaction stage from being stressed to developing an adaptation reaction to stress; a resistance stage during which resistance to stress is exerted by the adaptation reaction; and an exhaustion stage during which, due to prolonged stress load, the resistance becomes lost. In the present specification, a condition being at any stage of the stress reaction may be referred to as being in stress condition.

The stress condition in a subject can be confirmed by detecting the presence of the stress reaction, for example, by measuring the blood levels of adrenocortical hormones, such as cortisol, aldosterone, and androgen, whose secretion is known to be enhanced in response to stress. Furthermore, the present inventors revealed that stress enhances the expression of CCL5 at perivascular tissue in the boundary region of the third ventricle, thalamus, and dentate gyrus, and that stress triggers sympathetic activation in the paraventricular nucleus (PVN) (PCT/JP2018/007901 and Arima, Y. et al., eLife, 2017, 6: e25517). Accordingly, an increase in the expression level of CCL5 at the above-mentioned perivascular tissue and enhanced phosphorylation of cfos and CREB associated with sympathetic activation in the PVN can be also made use of as indicators to confirm whether to be in stress condition.

As described above, it is considered that stress load, especially, chronic stress load causes a stress reaction of a subject to be sustained over a long period of time, and as a result, inflammation is induced at intracerebral blood vessels of the subject, especially, blood vessels in the boundary region of the third ventricle, thalamus, and dentate gyrus, and thus, a disease or symptom, such as progressive multiple sclerosis, gastroenteritis, myocardial disorder, or sudden death, is induced. For example, a disease modeling mouse described in PCT/JP2018/007901 and Arima, Y. et al., eLife, 2017, 6: e25517 and prepared in the later-mentioned Examples exhibits continuous exacerbation of EAE pathological conditions, which reflects pathological conditions of progressive multiple sclerosis observed in subgroups of multiple sclerosis other than relapsing-remitting multiple sclerosis, that is, secondary progressive multiple sclerosis as the late phase of relapsing-remitting multiple sclerosis, primary progressive multiple sclerosis, and progressive-relapsing multiple sclerosis. In addition, the above-mentioned mouse exhibits pathological conditions of sudden death, which occurs within few days from when no EAE pathological condition is exhibited, pathological conditions of gastroenteritis including bleeding and inflammation in the stomach and upper small bowel, and pathological conditions of myocardial disorder including myocardial cell death or heart failure.

The pharmaceutical according to the present invention can be used for preventing and/or treating these diseases or symptoms, for example, progressive multiple sclerosis; gastroenteritis, such as gastritis, peptic ulcer, and inflammatory bowel diseases such as Crohn's disease and ulcerative colitis; sudden death; and myocardial disorder, such as angina, myocardial infarction, arrhythmia, cardiomyopathy, and heart failure. Preventing and/or treating used herein encompasses every type of medically acceptable preventive and/or therapeutic intervention intended, for example, for the cure, transient remission, or prevention of a disease or symptom. In other words, preventing and/or treating a disease or symptom encompasses medically acceptable intervention intended for various purposes, including the retardation or stop of progression of a disease or symptom, the regression or disappearance of lesion, the prevention of development, and the prevention of relapse.

An animal to which the pharmaceutical according to the present invention is to be administered is an animal having the problem of being affected with or developing these diseases or symptoms. Typical examples of the subject include rodents, such as mice, rats, hamsters, and guinea pigs; primates, such as human, chimpanzees, and rhesuses; livestock animals, such as pigs, cows, goats, horses, and sheep; and pet animals, such as dogs and cats. The subject is preferably human.

Another aspect of the present invention includes a method for preventing and/or treating at least one disease or symptom selected from the group consisting of progressive multiple sclerosis, gastroenteritis, myocardial disorder, and sudden death, the method including administering an effective amount of a substance that suppresses the expression of or inhibits the activity of at least one molecule selected from the group consisting of TMEM5, VSTM2L, C2CD4D, VSTM2A, LY6G6C, and ADRA2C, to a subject in need thereof.

The present invention will be further described in detail with Examples below, but the present invention is not limited to Examples.

### Examples

### Production of disease modeling mice

### (i) Stress load

PAWW stress load and induction of sleep disorder caused by the PAWW stress load were performed as previously reported (Miyazaki, K. et al., PloS one, 2013, 8, e55452). 6 to 8 week-old C57BL/6 mice (Japanese SLC) were individually bred and acclimated in respective plastic cages each having a running wheel. Then, through loading PAWW stress by replacing wood beddings in the cages with water having a depth of 1.5 cm to make the mice continuously exercise on the wheel for 2 days, sleep disorder was induced in the mice.

### (ii) Preparation of EAE-pathogenic CD4 positive T cells

Preparation and transfer of EAE-pathogenic CD4 positive T cells were performed as previously reported (Arima, Y. et al., Cell., 2012, 148, 447-457; Arima, Y. et al., eLife, 2015, 4, e08733; Ogura, H. et al., Immunity, 2008, 29, 628-636). Briefly, C57BL/6 mice were subcutaneously injected with a MOG (35-55) peptide (Sigma-Aldrich Co. LLC.) together with complete Freund's adjuvant (Sigma-Aldrich Co. LLC.) at the base of their tails, and then 0, 2, and 7 days after the MOG peptide injection, the mice were further intravenously injected with pertussis toxin (Sigma-Aldrich Co. LLC). 9 days after the MOG peptide injection, lymphocytes were collected from the spleens of the mice, and sorted using anti-CD4 microbeads (Miltenyi Biotec, Inc.) to obtain a cell population rich in CD4 positive T cells. This cell population (4 × 10⁶ cells) was co-cultured with MOG peptide-pulsed irradiated spleen cells (1 × 10⁷ cells) for 2 days in the presence of rIL-23 (10 ng/mL; R & D Systems, Inc). The cells were collected from a culture medium and CD4 positive T cells were enriched using the anti-CD4 microbeads to prepare EAE-pathogenic CD4 positive T cells.

### (iii) Transfer of EAE-Pathogenic CD4 Positive T Cells

2 days after starting the stress load described in the (i), the EAE-pathogenic CD4 positive T cells (1.5 × 10⁷ cells) prepared in the (ii) were transferred to the mice by intravenous injection to produce disease modeling mice.

### Example 1: Analysis of genes whose expression at specific cerebral blood vessels changes due to stress load

Approximately 100 frozen sections (15 µm in thickness) of the brains of C57BL/6 mice 2 days after the induction of sleep disorder were prepared, and fixed with PAXgene (QIAGEN Inc.) for 15 minutes, and subsequently fixed with 100% EtOH for 10 minutes. Tissues around the blood vessels in the third ventricular region in the sections were collected using a laser microdissection device, DM6000B (Leica Microsystems Inc.), and all RNAs were extracted using a RNeasy micro kit (QIAGEN Inc). RNA sequencing was conducted by Kazusa DNA Res. Inst., and expression analysis of all genes was performed.

As the result of the analysis, it was confirmed that the gene expression levels of TMEM5, VSTM2L, C2CD4D, VSTM2A, LY6G6C, and ADRA2C were increased due to stress load (Fig. 1 and Fig. 2), and the possibility that these factors might be involved in inflammation at the specific cerebral blood vessels and pathological conditions in the disease modeling mice was indicated.

### Example 2: Improvement of Pathological Conditions in Disease Modeling Mice by Antibody

Sleep disorder was induced in C57BL/6 mice, and EAE pathologic CD4 positive T cells were transferred into the mice. 5 days after the transfer, the head of each of the mice was fixed to a stereotaxic apparatus under anesthesia, fur over the skull was shaved, and the skin was cleaned with 70% ethanol. A 30-gauge needle was put into the specific blood vessels (coordinates: AP -1.06 mm; ML 1 mm; DV 2.25 mm), and 0.5 µl (liquid volume) of a rabbit anti-TMEM5 antibody (Novus (NB110-40445), Anti-TMEM5, rabbit Poly, 1.0 mg/ml), a mouse anti-VSTM2L antibody (Santa Cruz Biotechnology (SC-376538) Anti VSTM2L (A-4), 0.2 mg/ml), a rabbit anti-C2CD4D antibody (Bioss (bs-15144R), AntiC2CD4D Polyclonal Antibody, 1.0 mg/ml), a rabbit anti-VSTM2A antibody (Thermo Fisher (PA5-48140), VSTM2A Polyclonal Antibody), a rat anti-Ly6G6C antibody (clone; NIMP-R14, (NOVUS BIOLOGICALS), 0.1 mg/ml), a rabbit anti-alpha 2c Adrenergic Receptor antibody (GeneTex, 1 mg/ml), or a control antibody thereof (rat IgG (Sigma) or rabbit IgG (Sigma)) was microinjected thereinto over 90 seconds. Then, pathological conditions of EAE were evaluated with clinical scores. Clinical scores were determined as previously reported (Arima, Y. et al., Cell, 2012, 148, 447-457; Arima, Y. et al., eLife, 2015, 4, e08733; Ogura, H. et al., Immunity, 2008, 29, 628-636). A higher clinical score indicates that an encephalomyelitis symptom is severer. A clinical score of 0 is equivalent to a normal state without abnormal findings, while a clinical score of 5 is equivalent to death.

Fig. 3 and Fig. 4 illustrate changes over time of EAE clinical scores of mice to which the antibodies were respectively administered. A rapid increase in clinical score was observed in a control group to which a control antibody was administered, whereas few increase in clinical score was observed in any groups to which an anti-TMEM5 antibody, an anti-VSTM2L antibody, an antiC2CD4D antibody, an anti-VSTM2A antibody, an anti-LY6G6C antibody, or an anti-alpha 2c Adrenergic Receptor antibody was administered, and the progression of pathological conditions was significantly suppressed in the groups. Thus, it was confirmed that suppression, blockade, or inhibition of TMEM5, VSTM2L, C2CD4D, VSTM2A, LY6G6C, or ADRA2C contributes to preventing and/or treating pathological conditions in the disease modeling mice.

## Claims

1. A pharmaceutical for preventing and/or treating at least one disease or symptom selected from the group consisting of progressive multiple sclerosis, gastroenteritis, myocardial disorder, and sudden death,
the pharmaceutical containing, as an active ingredient, a substance that suppresses expression of or inhibits activity of at least one molecule selected from the group consisting of Transmembrane Protein 5 (TMEM5), V-Set And Transmembrane Domain Containing 2 Like (VSTM2L), C2 Calcium Dependent Domain Containing 4D (C2CD4D), and V-Set And Transmembrane Domain Containing 2A (VSTM2A),
the substance being at least one selected from the group consisting of a nucleic acid, an antibody, and an antibody derivative.

2. A pharmaceutical for preventing and/or treating at least one disease or symptom selected from the group consisting of progressive multiple sclerosis, gastroenteritis, myocardial disorder, and sudden death,
the pharmaceutical containing, as an active ingredient, a nucleic acid that suppresses expression of at least one molecule selected from the group consisting of lymphocyte antigen 6 family member G6C (LY6G6C) and an α2C adrenergic receptor (ADRA2C).

3. The pharmaceutical according to claim 1 or 2, wherein the disease or symptom is a disease or symptom caused by stress load.

4. A method for screening a substance capable of preventing and/or treating at least one disease or symptom selected from the group consisting of progressive multiple sclerosis, gastroenteritis, myocardial disorder, and sudden death, the method comprising the steps of:
(i) in a cell or animal that expresses at least one molecule selected from the group consisting of Transmembrane Protein 5 (TMEM5), V-Set And Transmembrane Domain Containing 2 Like (VSTM2L), C2 Calcium Dependent Domain Containing 4D (C2CD4D), V-Set And Transmembrane Domain Containing 2A (VSTM2A), lymphocyte antigen 6 family member G6C (LY6G6C), and an α2C adrenergic receptor (ADRA2C), measuring an expression level or activity of the molecule in presence and absence of a test substance; and
(ii) when the expression level or activity of the molecule in the presence of the test substance is lower or weaker than the expression level or activity of the molecule in the absence of the test substance, determining that the test substance has an ability to suppress the expression of or inhibit the activity of the molecule.
